# EUROPEAN PATENT APPLICATION

(11) **EP 4 792 875 A1**
(43) Date of publication of application: **19.08.2026**
(21) Application number: 25158705.1
(22) Date of filing: 18.02.2025
(51) Int. Cl.: A61N 5/06

(54) **DIRECTED UV LIGHT DEVICE**

(71) Applicant: Sunled Life Science B.V., 1098XG Amsterdam (NL)
(72) Inventor: FARSHADI, Aida, 1098 XG Amsterdam (NL); KRAMES, Michael, 1098 XG Amsterdam (NL); BERENDS, Anne, 1098 XG Amsterdam (NL)
(74) Representative: Hoyng Rokh Monegier B.V.

(57) **Abstract**

An electro-optical device (1) comprising a radiation unit (10) adapted to emit a radiation beam (11) having a peak emission wavelength in a range of 280-320 nm, a detection unit (20) adapted to detect a distance to a user and generate a detection signal based on the detection, and a radiation control unit (30) adapted to receive the detection signal and generate a control signal in response. The electro-optical device (1) is adapted to provide an irradiance in the range 0.003-0.5 mW/cm² to the user.

## Description

### TECHNICAL FIELD

The invention relates to an electro-optical device for providing ultraviolet radiation for health benefits in a user.

### BACKGROUND ART

The sunlight spectrum includes ultraviolet light at shorter wavelengths, typically divided into UV-C in the range 100 - 280 nm, UV-B at 280 - 320 nm and UV-A at 320 - 400 nm. Visible light is in the approximate range 380 - 650 nm, and infrared light at longer wavelengths including near-infrared in the range 780 - 1400 nm, with mid-infrared and far-infrared at still longer wavelengths.

It is well known that exposure to near-infrared (NIR, 780 - 1400 nm) light can be used to induce photobiomodulation (PBM) to bring health benefits to humans and animals. Ultraviolet light is another invisible component of sunlight that can induce beneficial effects in humans and animals, particularly UV-B light, as this has been found to induce the production of vitamin D, in particular vitamin D3, in humans and animals. Another health benefit related to UV-B exposure is an improved immune system. Moreover, UV-B exposure is also used as medical treatment for diseases like psoriasis and vitiligo. However, UV-B can also have a detrimental effect on health when an overdose is reached, such as skin cancer in extreme cases. Additionally, the eyes should be protected from too much exposure. For this reason, dose control is critically important, but this has obtained limited attention in current (product) literature.

Another important element of designing devices that bring the health benefits of UV-B to the user is energy efficiency. Although the UV-B dose needed for health benefits is low (much lower than the NIR dose needed to induce PBM), it is nevertheless undesirable from an energy perspective to radiate invisible light to empty spaces in which no user is present. Targeting the UV-B light at the user is important for energy effective solutions and overlooked in current (product) literature.

### SUMMARY OF THE INVENTION

It is an object of the present invention to address these problems and provide a means to induce the production of vitamin D3 in a user by delivering UV-B radiation via free space to the user in a controlled and targeted manner. It is a further object of the invention to deliver the stimulus to vitamin production in a manner which is cost effective and energy efficient. It is a further object of the invention to monitor and control a UV-B dose delivered to a user through real-time detection and adjustment. Implementations particularly suitable for carrying out the invention include those where the location of the user is known and/or can be monitored. This information provides an opportunity for the delivery of the UV-B radiation at a dose sufficient to induce vitamin D3 production in the user without exceeding safe limits while maintaining optimal energy usage. Information regarding the location of the user also includes the length of time that the user is present in the irradiated area, so that a dose may be calculated and monitored.

According to a first aspect of the invention, there is provided an electro-optical device comprising a radiation unit adapted to emit a radiation beam having a peak emission wavelength in a range of 280-320 nm, a detection unit adapted to detect a distance to a user and generate a detection signal based on the detection, and a radiation control unit adapted to receive the detection signal and generate a control signal in response, wherein the electro-optical device is adapted to provide an irradiance in the range 0.003-0.5 mW/cm² to the user.

The wavelength range from 280 nm to 320 nm encompasses ultraviolet light in the UV-B range, which is particularly suited for stimulating the production of vitamin D3 in humans and animals. The electro-optical device may provide a dose of UV-B radiation to the user in the range 0.1-200 mJ/cm², preferably in the range 1-100 mJ/cm², and more preferably in the range 3-50 mJ/cm². The radiation control unit is preferably adapted to adjust the radiant intensity and/or an on-time of the radiation beam emitted by the electro-optical device to provide a predetermined dose to the user, and preferably to avoid exceeding a predetermined dose.

The electro-optical device is preferably designed to provide a narrow radiation beam that is directed to the user, preferably directed to predetermined part of the user such as the user's face, neck, hands and/or forearms of the user, to irradiate the user without substantially spreading the beam elsewhere. The electro-optical device is preferably adapted to project the radiation beam in a narrow spread angle within +/- 30°, preferably within +/- 25°, more preferably within +/- 20°, more preferably within +/- 15° about a center line of the radiation beam. The electro-optical device is preferably adapted to adjust the spread angle of the radiation beam depending on the control signal from the radiation control unit. This enables the electro-optical device to make the radiation beam narrower or wider depending on the distance to the user and the size of the predetermined part of the user (e.g. face or hands) facing towards the electro-optical device, so that the beam irradiates substantially all of the desired part of the user but without irradiating elsewhere.

The detection unit is preferably adapted to detect a direction of the user with respect to the electro-optical device, and the detection signal preferably includes an indication of the detected direction to enable the electro-optical device to direct the radiation beam towards the user (and a predetermined part of the user) in dependence on the detected direction.

The detection unit is preferably adapted to detect a predetermined part of the user and include an indication of the detected predetermined part in the detection signal. The predetermined part may cover a substantial portion of the face, neck, hands, and/or forearms of the user. The electro-optical device is preferably adapted to adjust a direction, radiation pattern, radiant intensity, and/or on-time of the radiation beam in dependence on the control signal.

The predetermined part of the user may include the eyes of the user, and the electro-optical device is preferably adapted to reduce a radiant intensity of the radiation beam if the detection signal indicates that the radiation beam is directed to a predetermined part of the user that includes at least a portion of the user's eyes. The electro-optical device may be adapted to adjust a direction or a radiation pattern of the radiation beam to reduce a radiant intensity of the radiation beam in a direction of the user's eyes if the detection signal indicates that the radiation beam is directed to a predetermined part of the user that includes at least a portion of the user's eyes.

The electro-optical device may be adapted to adjust a radiation pattern of the radiation beam by adjusting a direction, spread angle, and/or the radiant intensity of portions of the radiation beam. The radiation unit preferably comprises an optical element comprising one or more lenses, mirrors, diffraction optical elements, filtering elements, absorptive coatings, and the radiation control unit may be adapted to move and/or rotate a part of the optical element to adjust a direction of the radiation beam.

The radiation unit preferably comprises a plurality of radiation elements, each driven by a driving current, and the radiation control unit may be adapted to adjust the driving current for individual radiation elements to adjust the direction, spread angle, radiation pattern, and/or the radiant intensity of the radiation beam. The radiation unit preferably comprises a plurality of LEDs, EELDs and/or VCSELs, and the radiation control unit may be adapted to switch on or switch off a subset of the plurality of LEDs, EELDs and/or VCSELs to adjust the direction, spread angle, radiation pattern, and/or the radiant intensity of the radiation beam.

The detection unit is preferably adapted to distinguish between individual users, and the detection signal may include an indication of the detected individual user so that the electro-optical device can adjust a direction, radiation pattern, radiant intensity, and/or on-time of the radiation beam in dependence on the detected individual user. The electro-optical device may be adapted to adjust a dose provided to the user by the radiation beam in dependence on detected identity of the user.

The detection unit may be adapted to detect a characteristic of the user such as skin type of the user, such as the pigmentation, age, or other characteristics of the user's skin. The electro-optical device may be adapted to adjust the dose provided to the user by the radiation beam based on one or more properties of the skin of the user.

The electro-optical device preferably comprises a second radiation unit adapted to emit a second radiation beam having a peak emission wavelength in a range of 610-1400 nm, the electro-optical device being adapted to control the second radiation beam based on the control signal to provide a peak irradiation intensity above 0.1 mW/cm² to the user.

The electro-optical device may be implemented in many different forms, such a device adapted to be placed on a desk or a table, and may be incorporated in a portable user equipment and/or a display apparatus and/or a luminaire.

### BRIEF DESCRIPTION OF THE DRAWINGS

Embodiments will now be described, by way of example only, with reference to the accompanying schematic drawings in which corresponding reference symbols indicate corresponding parts, and in which:
Fig. 1 is a schematic diagram of an embodiment of the electro-optical device 1 according to the present invention;
Fig. 2 is a plot of an action spectrum and dose limit curve;
Figs. 3A-3F are schematic diagrams of various devices in which the electro-optical device 1 according to the present invention may be implemented;
Figs. 4A-4D are schematic diagrams of embodiments of a radiation unit for use in the electro-optical device of Fig. 1;
Figs. 5A-5C are schematic diagrams of details of further embodiments of a radiation unit for use in the electro-optical device of Fig. 1;
Fig. 6 is a schematic diagram of an embodiment of an electro-optical device 1 comprising a timer for dose control;
Fig. 7 is a schematic diagram of an embodiment of the electro-optical device 1 capable of recognizing and distinguishing between individual users; and
Fig. 8 is a schematic diagram of an embodiment of the electro-optical device 1 including a second radiation unit for generating a near-infrared radiation beam.

### DESCRIPTION OF ILLUSTRATIVE EMBODIMENTS

The following is a description of certain embodiments of the invention, given by way of example only and with reference to the drawings.

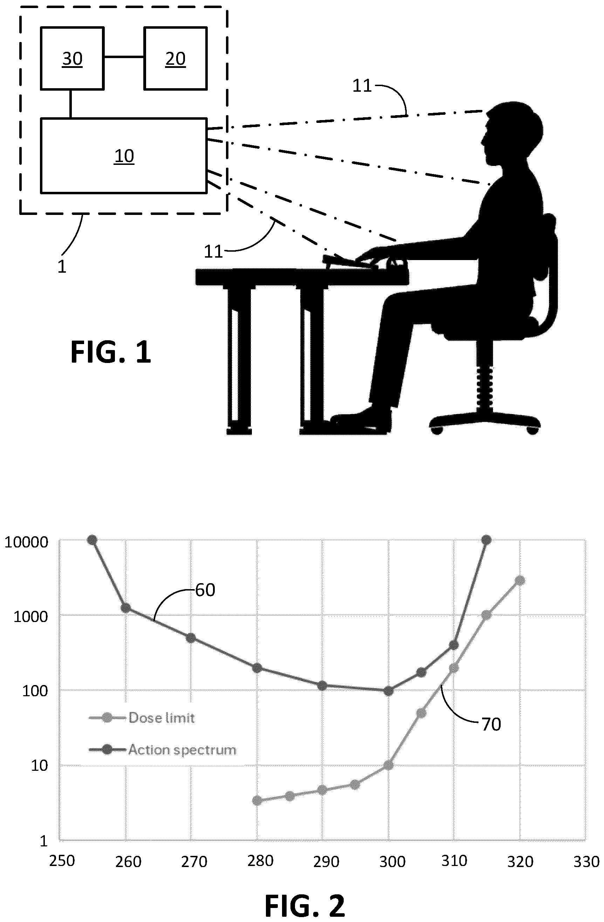

Fig. 1 shows an embodiment of an electro-optical device 1 according to the present invention. The device 1 comprises a radiation unit 10 adapted to generate a radiation beam 11, a detection unit 20 adapted to detect a user and generate a detection signal based on the detection, and a radiation control unit 30 adapted to receive the detection signal and generate a control signal in response. The electro-optical device 1 is adapted to control the radiation beam based on the control signal to provide a dose in a range of 0.1-200 mJ/cm² to the user.

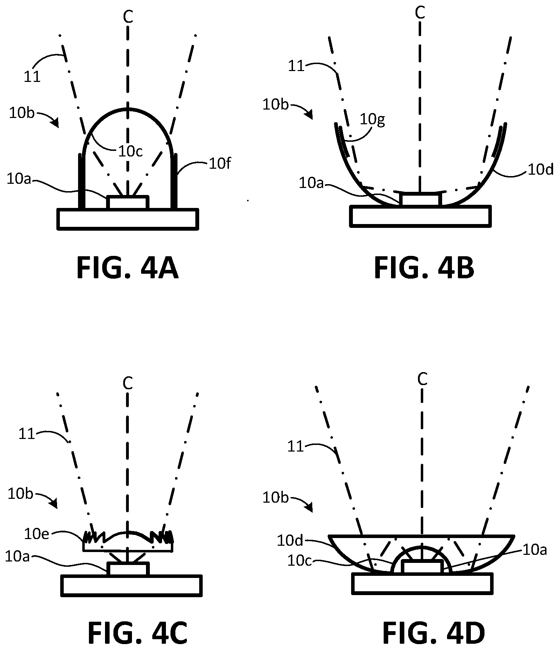

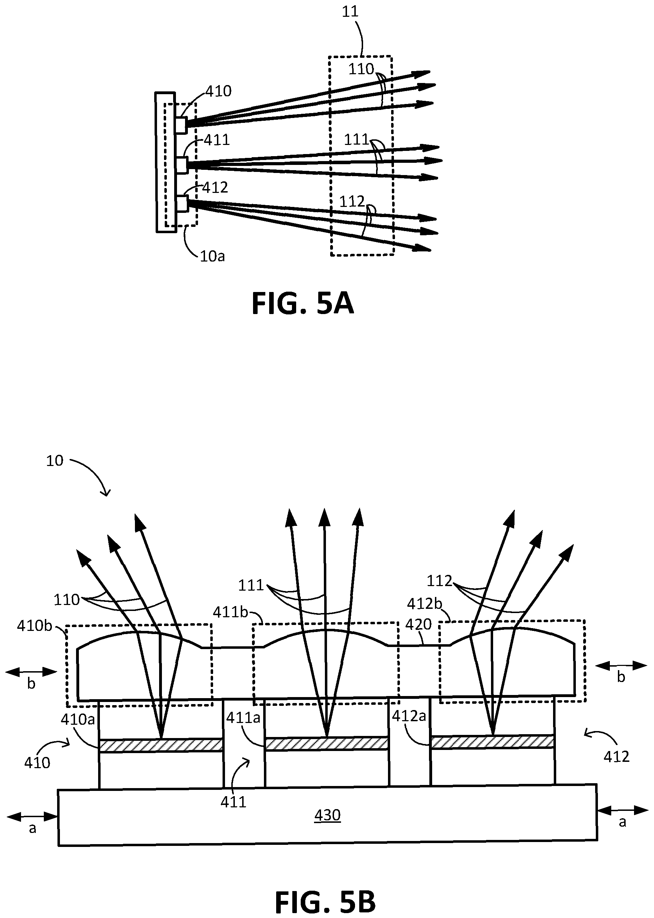

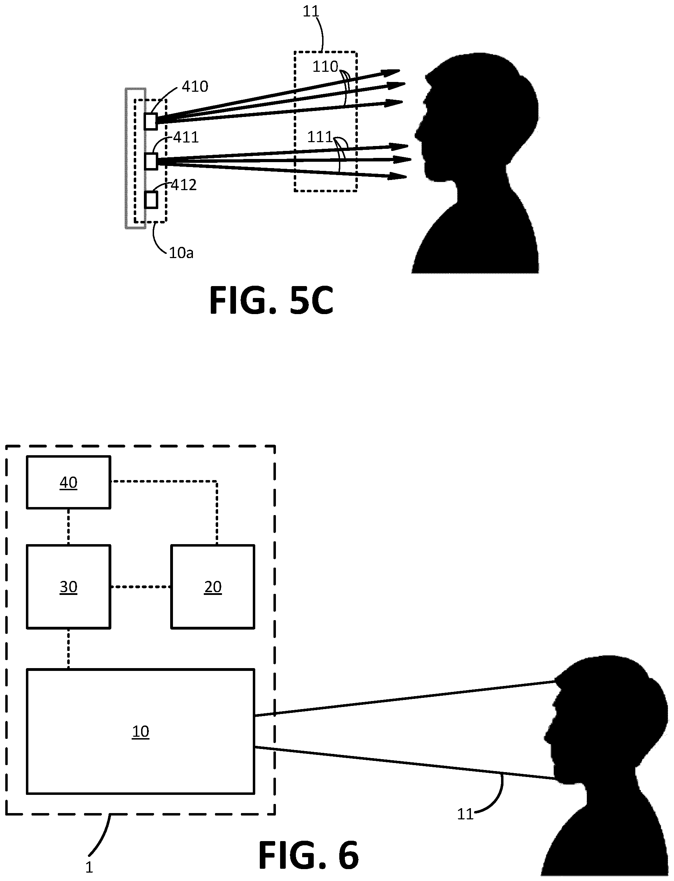

The radiation unit 10 comprises one or more radiation elements, such as light emitting diodes (LEDs), edge-emitting laser diodes (EELDs) and/or vertical-cavity surface emitting lasers (VCSELs) to generate the radiation beam 11. In a preferred embodiment, the radiation unit 10 generates the radiation beam 11 using an array of LEDs, EELDs and/or VCSELs. The radiation unit 10 may comprise one or more optical elements, such as lenses, mirrors, and/or diffractive optical elements (DOEs), as described below in relation to Figs. 4 and 5, to adjust the direction, spread angle and other characteristics of the radiation beam 11.

The detection unit 20 functions to detect the distance to the user, i.e. the distance between the electro-optical device 1 and the user, or the distance to a predetermined part of the body of the user. The predetermined part may relate to any part of the user's body. In a preferred embodiment, the predetermined part covers at least a portion of the face and/or neck of the user, and alternatively or additionally a substantial portion of the hands and optionally the forearms of the user, as shown in Fig. 1. These parts of the user are usually not covered (or not completely covered) by clothing and thus can be conveniently irradiated by the radiation beam 11. In a preferred embodiment, the predetermined part excludes the user's eyes, as they are more sensitive to UV-B radiation and the dose provided by the electro-optical device 1 to the user may be higher when the user's eyes are not directly exposed to the radiation beam 11.

The detection unit 20 may also function to detect the position and/or orientation of the user with respect to the electro-optical device 1, and may also detect the position and/or orientation of the predetermined part of the user (e.g. the user's face or hands).

The detection unit 20 may include one or more emitters and receivers for emitting radiation and receiving radiation reflected from the user in order to detect the distance, position and/or orientation of the user, and detect specific parts of the user such as the user's face and hands. For example, the detection unit 20 may use radar technology for user detection, or near-infrared emitters and sensors as used in vehicle driver detection systems, a time-of-flight sensor, or a camera-based sensor using image analysis.

The detection unit 20 may additionally include image sensing technology with facial recognition capabilities, to enable detection of the distance, position and orientation of the user's face with respect to the electro-optical device 1. The detection unit 20 may be configured to recognize a human face, and may be additionally configured to recognize the separate features of a face. In particular, the detection unit 20 may be configured to determine the position of the user's eyes within the area of the user's face, to enable the radiation beam 11 to be directed towards the user's face while avoiding directing the beam into the user's eyes.

The detection unit 20 may be additionally configured to recognize a particular person's face, i.e. to differentiate between different users. This technology is included in many devices, such as smart phones, and enables the electro-optical device to monitor and control the dose of UV-B provided to an individual user.

The detection unit 20 generates a detection signal that provides an indication of the detected distance to the user or predetermined part of the user. Where the detection unit 10 detects other characteristics of the user, such as the position and orientation of the user or predetermined part of the user, or facial recognition information of the user, this information is also indicated in the detection signal.

The radiation control unit 30 receives the detection signal generated by the detection unit 20. The radiation control unit 30 generates a control signal in response to the detection signal, the control signal being an input to the radiation unit 10 and optionally other components of the electro-optical device 1, for control of the radiation beam 11. The radiation control unit 30 may be implemented using hardware circuits or software or firmware executing on a processor, or a combination of hardware and software.

The radiation control unit 30 functions to direct the radiation beam 11 towards the user and adjust beam to administer the appropriate dose of the UV-B radiation to the user. This is achieved by adjusting one or more characteristics of the radiation beam 11, these characteristics including the direction in which the beam is emitted from the electro-optical device, the spread angle of the emitted beam, the focusing of the beam, the radiation pattern of the beam, the radiant intensity of the beam, the on-time and off time of the beam or sub-beams forming the beam, and/or other relevant characteristics.

These adjustments to the radiation beam 11 may be made to adjust the beam characteristics to achieve one or more objectives, such as directing the beam towards a user detected by the detection unit 20, to focus or direct the beam towards a particular part of the user or to avoid a particular part of the user (such as the user's eyes), and/or to adjust the radiant intensity and/or on-time of the beam to provide a certain irradiance or dose to the user.

The direction of the radiation beam 11 may be adjusted to direct the beam towards a user, where the distance, position and/or orientation of the user has been detected by the detection unit 20. Thus, when the user moves with respect to the electro-optical device 1, the direction of the beam may be adjusted so that the beam remains pointed towards the user and pointed towards the predetermined part of the user.

The spread-angle of the radiation beam 11 may also be adjusted depending on the distance, position and/or orientation of the user, e.g. increasing the spread-angle when the user is closer to the electro-optical device 1 and reducing the spread-angle when the user is further away, and adjusting the spread angle of the beam in dependence on the size of the predetermined part of the user to be irradiated.

The radiation pattern of the radiation beam 11 may also be adjusted, e.g. to irradiate a particular part of the user more precisely, such as irradiating the user's face while avoiding directing the radiation beam 11 directly towards the user's eyes. The radiation pattern or shape of the radiation beam 11 may be adjusted, for example, by switching on or off individual sub-beams which make up the radiation beam 11, or adjusting the radiant intensity of individual sub-beams. Examples of various means for adjusting the direction, spread angle and radiation pattern of the radiation beam 11 are described herein in relation to Figs. 4 and 5.

The radiant intensity of the radiation beam 11 may be adjusted to provide a certain irradiance to the user (e.g. to achieve a certain dose to the user over a certain time period). This may be done by adjusting the electrical driving current supplied to the radiation elements of the radiation unit 10. The driving current may be varied in amplitude, pulsed on and off or otherwise modulated to adjust the radiant intensity of the radiation beam 11 emitted by the electro-optical device. For embodiments employing an array of radiation elements, the driving current supplied to each radiation element may be individually controlled to achieve variation of the radiant intensity of the radiation beam 11, e.g. by switching off or modulating or pulsing the driving current supplied to a subset of the radiation elements.

The radiation control unit 30 may include a dose monitoring system, which calculates and monitors the dose of UV-B radiation provided to the user. Dose is typically expressed as the unit of the accumulated energy per square centimeter, e.g., mJ/cm², or as a total dose value expressed as energy in kJ. The dose may also be expressed as a standard erythema dose (SED), where 1 SED = 10 mJ/cm² at 298 nm.

The dose may be calculated based on the distance to the user detected by the detector unit 20, the time period in which the user was present and irradiated by the radiation beam 11, and the radiant intensity of the radiation beam 11 while the user was irradiated. Other characteristics of the radiation beam 11 that affect the dose delivered may also be taken into account to calculate dose, such as spread-angle of the beam.

Fig. 2 shows an "action spectrum" 60 and a dose limit curve 70, plotted with vertical axis of dose on the user's skin measured in mJ/cm² and horizontal axis showing the wavelength of the light in nanometers (nm).

The action spectrum 60 provides an indication of the relative effectiveness of light of a particular wavelength for inducing vitamin D3 production in a user. The action spectrum 10 indicates that a relatively low dose is needed for inducing vitamin D3 production in the range 280-310 nm (i.e. in the UV-B region) while a much higher dose is needed at wavelengths lower than 280 nm and higher than 310 nm. For example, the lowest point of the action spectrum 60 occurs at or around 300 nm, indicating the most effective wavelength for vitamin D3 production. At this wavelength, a dose of only 100 mJ/cm² is required to induce the same amount of vitamin D3 production as a much higher dose of 1000 mJ/cm² at 260 nm. Note that the action spectrum 60 is normalized to a certain level of vitamin D3 production in a representative user, not to the ideal or minimum amount of vitamin D3 production. In practice, lower doses than indicated by the action spectrum 60 may be used to induce beneficial amounts of vitamin D3 production. A dose of UV-B radiation in the range 0.1-200 mJ/cm² may be used, preferably 1-100 mJ/cm², more preferably 3-50 mJ/cm².

The dose limit curve 70 shown in Fig. 2 is derived from guidelines issued by the ICNIRP (International Commission on Non-Ionizing Radiation Protection). The dose limit curve 70 indicates the maximum dose for light wavelengths between 280-320 nm to which a user's eyes should be exposed, to avoid damage to the eye. Dose limits related to other health risks from exposure of skin to UV-B radiation are less well defined as they depend on individual skin characteristics, which can vary widely among users.

By plotting both the action spectrum 60 and dose limit 70 together in the same graph, the range of wavelengths that are most suited for implementation of the electro-optical device 1 can be readily determined. In particular, the UV-B wavelengths that are most conducive to the production of vitamin D3 in a user can be readily compared to the UV-B wavelengths at which higher doses are possible without undue risk of exposure to a user's eyes.

The ideal dose for production of vitamin D3 depends on a number of variables, including the baseline concentration of vitamin D3 (or a precursor such as 25(OH)D3) in the user, the skin characteristics of the user, and the area (in cm²) of the user exposed to the UV-B radiation. Studies have found that a dose in the range 3.75-30 mJ/cm² at 298 nm can be enough to induce significant and sufficient vitamin D3 production in some users.

Table 1 below indicates for various wavelengths an effective dose range, the maximum dose taking into account the eye safety limit when the user's eyes may be exposed to the UV-B radiation (Dose 1), and a dose comparable to 20 SED (allowable when the eye safety limit is not applicable because the user's eyes are not directly exposed to the UV-B radiation, Dose 2).

**TABLE 1**

| Wavelength | Dose 1 (mJ/cm²) | Dose 2 (mJ/cm²) |
|---|---|---|
| 300 | 10 | 200 |
| 305 | 50 | 350 |
| 310 | 200 | 808 |

For comparison, the irradiance and dosing time from natural sunlight are listed in Table 2 below. The values for irradiance are estimated values at the wavelengths indicated, for a typical sunny summer day in the Netherlands. The exposure time to achieve the maximum dose taking into account the eye safety limit (Dose 1) and a dose comparable to 20 SED (Dose 2).

**TABLE 2**

| Wavelength (nm) | Irradiance (mW/cm²) | Dose 1 (mJ/cm²) | Dose time 1 (hours) | Dose 2 (mJ/cm²) | Dose time 2 (hours) |
|---|---|---|---|---|---|
| 305 | 0.0046 | 50 | 1.46 | 348 | 21.0 |
| 310 | 0.0095 | 200 | 5.84 | 808 | 23.6 |

The electro-optical device 1 may be designed to provide the UV-B radiation to user at a higher irradiance than sunlight, to reduce the time to achieve an effective dose. The radiation unit 10 preferably provides the radiation beam 11 at an irradiance of between 0.003 mW/cm² and 0.5 mW/cm² to the user. An indication of the exposure time at which a target dose is reached at an irradiance of 0.003 mW/cm² and at a higher irradiance of 0.5 mW/cm² is provided below in Table 3 (for doses taking into account the eye safety limit) and Table 4 (for doses when the eye safety limit is not applicable, comparable to 20 SED).

**TABLE 3**

| Wavelength (nm) | Target dose (mJ/cm²) | Irradiance (mW/cm²) | Time to dose | Irradiance (mW/cm²) | Time to dose |
|---|---|---|---|---|---|
| 300 | 10 | 0.003 | 0.9 hrs | 0.5 | 0.3 min |
| 305 | 50 | 0.003 | 4.4 hrs | 0.5 | 1.67 min |
| 310 | 200 | 0.003 | 18 hrs | 0.5 | 6.7 min |

**TABLE 4**

| Wavelength (nm) | Target dose (mJ/cm²) | Irradiance (mW/cm²) | Time to dose | Irradiance (mW/cm²) | Time to dose |
|---|---|---|---|---|---|
| 300 | 200 | 0.003 | 17.7 hrs | 0.5 | 6.7 min |
| 305 | 348 | 0.003 | 31 hrs | 0.5 | 11 min |
| 310 | 808 | 0.003 | 72 hrs | 0.5 | 27 min |

The radiation control unit 30 may be programmed to calculate the dose received by the user and control the radiation unit 10 to avoid exceeding the dose values indicated in the tables above. Furthermore, studies suggest that the body has a natural balancing mechanism to prevent vitamin D3 intoxication. After prolonged exposure and/or exposure of large surface areas of a user's body, the concentration of 25(OH)D3 (a precursor to vitamin D3) in a user saturates. This saturation effect allows to study the effect of dose in combination with exposed surface area, skin characteristics, etc.

An effective dose can be provided by exposing a small area of a user's body. Studies show that exposure of a body surface area as small as 10% of a user's body to a dose of 10 mJ/cm² is already enough to generate or maintain a healthy vitamin D3 status. At lower doses, the amount of 25(OH)D3 produced depends on the exposed surface area (one study used a dose of 7.5 mJ/cm² on 6% 12% or 24% of the user's body), while for higher doses (10 or 30 mJ/cm²) the exposed surface area was not a significant factor in determining how much 25(OH)D3 was produced. This suggests that exposing a user's face and neck (about 9% of total body area) with a dose of 10-30 mJ/cm² is sufficient to induce a healthy amount of 25(OH)D3 (and thus vitamin D3).

However, it should be noted that variations among users, including skin pigmentation and aged skin, may reduce the rate of vitamin D3 production and amount produced. These individual variations in skin type or age may be addressed by incorporating sensing technology in the detector unit 20 that is able to detect these characteristics of the user, e.g. a camera and processor for image analysis. The radiation control unit 30 may also include logic to determine an optimum dose for an individual user based on detection of these characteristics, and adjust the radiation beam 11 accordingly.

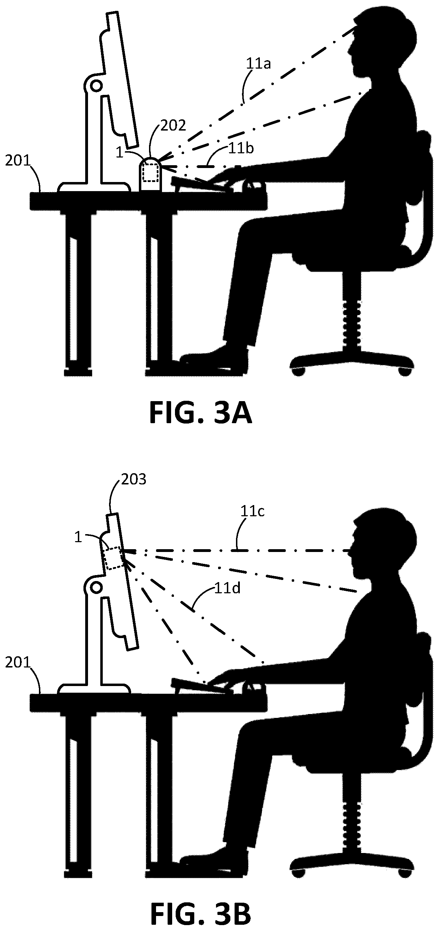

Fig. 3A-3F show various examples of how the electro-optical device 1 may be implemented. Such devices may contain the features and functions described herein with respect to Figs. 1 and 2, and the features and functions of radiation units the features and functions described herein with respect to Figs. 4A-4D and 5A-5B.

Fig. 3A shows an embodiment in which the electro-optical device 1 is designed to be placed on a desk or table 201, such as in the form of a desk accessory 202, e.g. a small USB powered device. A desk or table has well-defined dimensions. This makes it an ideal place to locate the electro-optical device 1 as these dimensions set the boundary of the distance between the electro-optical device 1 and the user. Taking these dimensions into account, this makes it possible to design the radiation unit 10 to generate a radiation beam 11 having the desired characteristics (e.g. spreading angle, beam direction, radiant intensity etc.) so that the beam is directed to the user or a predetermined part of the user and the width of the beam is just enough to cover the predetermined area, with sufficient power to induce the production of vitamin D3 in the user. Many people have objects on their desk (e.g. pencil holder, sound speakers, etc.). These objects are suitable for incorporating the electro-optical device 1 which may be relatively small, and are typically placed relatively close to the user (particularly the user's hands) increasing the efficiency of irradiating the user.

In the embodiment shown in Fig. 1, the electro-optical device generates a radiation beam 11a directed towards the face and neck of the user, and additionally or alternatively, generates a radiation beam 11b directed to the hands of the user. The user's eyes will be exposed to radiation beam 11a (unless the radiation pattern of the beam is adjusted to avoid the eyes or other measures are taken to avoid exposure) whereas radiation beam 11b is directed away from the user's eyes. This allows the radiant intensity of radiation beam 11b to be set at a value to provide a relatively higher dose on the user's hands, in comparison to the radiant intensity of radiation beam 11a which should be set at a value to provide a relatively lower dose on the user's face, in particular in the area of the user's eyes.

Fig. 3B shows an embodiment in which the electro-optical device 1 is embodied in a computer display 203. Similar to Fig. 3A, this embodiment enables the typical dimensions of a desk or table 201 to be utilized to design the electro-optical device 1 so that the irradiance of the radiation beams 11c and 11d is high enough to induce the production of vitamin D3 in the user within a convenient exposure period, but is not too high. In the embodiment shown, the radiation beam 11c is directed to a lower portion of the user's face to avoid direct exposure of the user's eyes to the beam, and/or radiation beam 11d is directed to the user's hands and forearms. These radiation beams 11c, 11d avoid direct exposure of the user's eyes to the radiation. This allows a higher dose of UV-B radiation to be administered to the user to efficiently induce vitamin D3 production in a shorter exposure time, while avoiding harmful effects on the user's eyes.

Similarly, the electro-optical device 1 may be embodied in a television (not shown), where the user may be assumed to sit at a certain distance from the television screen. Also, the radiation unit may be incorporated into a separate devices that mount onto or nearby the computer display or television or similar monitor.

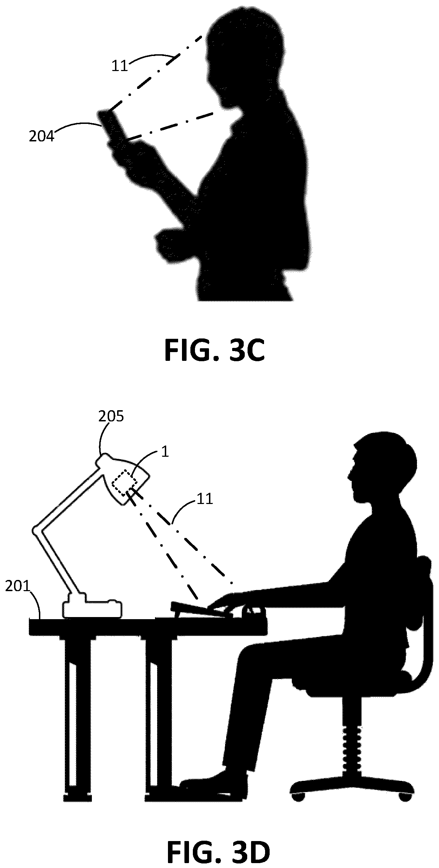

Fig. 3C shows an embodiment in which the electro-optical device 1 is incorporated in a portable user equipment 204, such as a smartphone. This embodiment has several advantages. First, a detection unit 10 for detecting a distance to the user is usually already implemented in such devices which reduces the cost of implementing the present invention, and the detection unit often includes facial detection and user recognition functionality. Moreover, many users have a relatively long screentime with such devices and keep a relatively short and stable distance between the electro-optical device 1 in the screen of the device and the user's face (e.g. 30 cm).

Fig. 3D shows an embodiment in which the electro-optical device 1 is incorporated in a desk or table lamp 205. This embodiment is particularly suited for directing the radiation beam 11 onto the user's hands and/or forearms at a relatively short and stable distance between the electro-optical device 1 and the user's hands, depending on the size of the desk or table lamp.

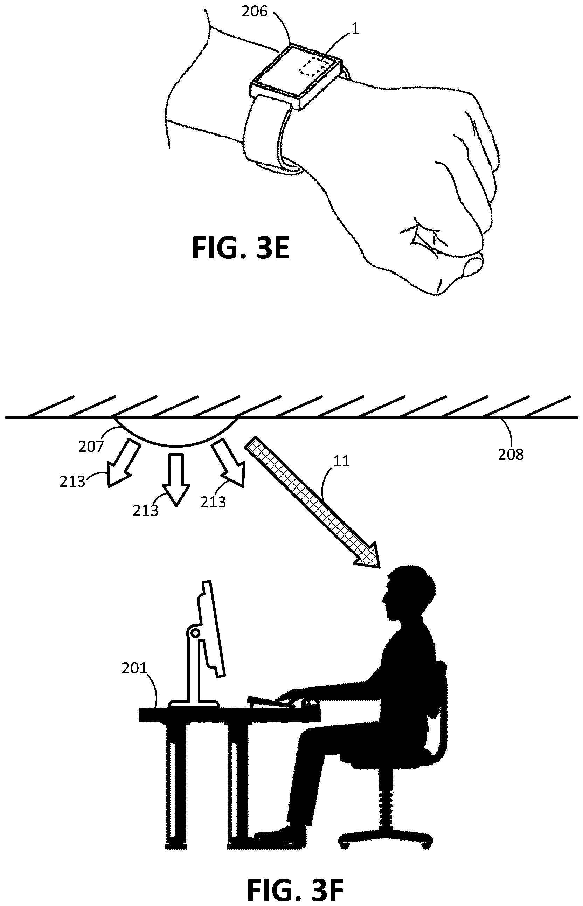

Fig. 3E shows an embodiment in which the electro-optical device 1 is incorporated in a wearable device 206, such as a wrist watch. This embodiment may be used for directing a radiation beam 11 upwards onto the user's face and neck, and may also direct a radiation beam 11 laterally onto the user's hands and/or forearms, at a relatively short and stable distance between the electro-optical device 1 and the user's face/neck and the user's hands/forearms.

Fig. 3F shows an embodiment in which the electro-optical device 1 is incorporated in a general lighting apparatus 207. In the embodiment shown, the general lighting apparatus 207 is mounted on a ceiling 208. It may also be mounted on a wall, or as a self standing lamp, or any other lamp arrangement. In addition to the radiation beam 11, the general lighting apparatus 207 additionally emits visible light 213 for the purpose of general lighting, as described in WO 2020/119965 and WO 2021/099642.

In contrast to the prior art, the present invention allows the radiation beam 11 to be much more directed and/or focused. Preferably, the radiation beam 11 is focused onto particular parts of the user while avoiding spreading the beam elsewhere. To accomplish this, the radiation beam 11 preferably has a spread angle within 2×30°, preferably within 2×25°, more preferably within 2×20°, more preferably within 2×15°. As can be seen in Fig. 3F, while the visible light 213 spreads in a wide angle for the general lighting function, the radiation beam 11 is projected towards the user, and onto a predetermined part of the user (e.g. user's face and/or hands) so as to induce production of vitamin D3. This allows energy saving and optimal dosing. The better the delivery of the radiation beam 11 is controlled, the less energy is `lost' so that the user receives exactly the right amount of UV-B radiation.

### Radiation adjustment

Figs. 4A-4D show several embodiments of the radiation unit 10 in which the radiation pattern is controlled using an optical element 10b. These measures may be implemented in the electro-optical device 1 described herein and in the embodiments shown in Figs. 1, 2 and 3A-3E.

In these embodiments, the radiation unit 10 comprises one or more radiation elements 10a and may optionally comprise an optical element 10b for each radiation element (or shared by multiple radiation elements). The optical element 10b may comprise one or more lenses 10c (Fig. 4A), one or more mirrors 10d (Fig. 4B), one or more diffractive optical elements 10e (DOEs) (Fig. 4C), or a combination of these. For example, one or more lenses 10c and one or more mirrors 10d may be combined to utilize total internal reflection (Fig. 4D). Other combinations are also possible, including combinations of the same type of deflection element (e.g. two or more lenses, two or more mirrors, two or more DOEs). The radiation pattern may be adjusted by moving and/or rotating a part of the optical element, e.g. moving one lens, mirror or DOE while maintaining the position of another lens, mirror or DOE.

In this way, the propagation of the radiation beam 11 may be adjusted in various ways. For example, the radiation control unit 30 may be adapted to adjust the direction (along the centerline 'C') and/or spread angle of the radiation beam and/or the focus of the beam, in reaction to an input from the detection unit 10.

Alternatively or additionally, the radiation pattern may be adjusted by altering the radiation emitted from the radiation elements 10a, as shown in Figs. 5A and 5B, which may be implemented in the embodiments discussed above.

The optical element 10b may additionally comprise one or more filtering elements, such as optical filters 10f, or absorptive coatings 10g, positioned to prevent scattered and reflected UV radiation from escaping outside the intended beam path. These filtering elements enhance safety by minimizing uncontrolled UV leakage. The filtering elements may be integrated with the lenses, mirrors, and/or diffractive optical elements, or may be implemented as separate components within the optical element 10b.

As shown in Fig. 5A, a multiple-element emitter may be used to generate the radiation beam 11. In the embodiment shown, the radiation unit 10 comprises a plurality of radiation elements 410, 411, 412, each driven by a driving current. Although only three radiation elements are shown, any other number for the radiation elements may be implemented. In this embodiment, the radiation control unit 30 (not shown in Fig. 4) is adapted to adjust the driving current for individual radiation elements in reaction to an input from the detection unit 20, to adjust the radiation pattern of the radiation beam 11 (which in this embodiment is depicted as the collection of radiation sub-beams 110, 111, 112 from the individual radiation elements 410, 411, 412).

Each radiation element 410, 411, 412 in the multiple-element emitter may be arranged to map to an effective illuminated region. For example, when the effective illuminated region for an individual radiation emitter falls within the predetermined part of the user (i.e. a portion of the user desired to be exposed to radiation beam 11), that radiation element is switched on. When the effective illuminated region for an individual radiation emitter is not within the predetermined part of the user, that radiation element is not switched on (e.g. switched off). This enables the electro-optical device 1 to adjust the radiation pattern of the radiation beam 11 to expose the desired parts of the user (e.g. exposing the user's face but not the user's eyes) with precision and without requiring any moving parts.

In an embodiment, the radiation unit comprises a plurality of LEDs, and the radiation control unit is adapted to switch on or switch off a subset of the plurality of LEDs in reaction to an input from the detection unit, so as to adjust the radiation pattern of the radiation beam. This enables switching on and switching off subset of the plurality of LEDs based on the detection of the predetermined part of the user. Each subset of LEDs may comprise one or more LEDs, and different subsets may comprise different numbers of LEDs. Different subsets of LEDs may be used for projecting the radiation beam towards different users.

Lasers such as edge-emitting laser diodes (EELDs) and Vertical-cavity surface emitting lasers (VCSELs) and EELD and VCSEL arrays may also be used in the radiation unit 10. Compared to LEDs, EELD and VCSEL arrays have an advantage of producing a relatively narrow radiation beams.

An array of radiation elements typically contains a plurality of individual emitters. With VCSELs it is possible to create a very well defined beam spot with sharp edges. Each individual emitter may be provided with an integrated deflection member such as mirror, lens or DOE, to set a beam direction (similar to the arrangements shown in Figs. 4A-4D). Typically, each deflection member is fixed in position but configured differently for each individual emitter, so the sub-beam from each emitter is directed in a different predetermined direction. Using this arrangement, the individual emitters can be switched on or off to generate a radiation beam 11 having a desired radiation pattern, to irradiate a desired predetermined part of the user (e.g. irradiating the user's face but avoiding irradiating the user's eyes). In this way, a radiation unit 10 may incorporate an array of these emitters, and the radiation control unit 30 can generate a control signal to switch on and off the individual emitters based on the detection signal from the detection unit 20, to irradiate a predetermined part of the user. An optical diffusor element (between the VCSEL emitter and the user's eye) may also be include, to break up the coherence of the laser and increase the effective source size, reducing eye safety hazard.

Fig. 5B shows an embodiment of the radiation unit 10 using a VCSEL array comprising a plurality of radiation elements/emitters 410, 411, 412. This arrangement may also be used for an LED or EELD array. In the embodiment shown, the VCSEL array has a plurality of active regions 410a, 411a, 412a (typically in the form of quantum wells in a semiconductor laser diode) for generating radiation sub-beams, which may function as the radiation element(s) 10a. A substrate 420 forming a plurality of microlenses 410b, 411b, 412b may function as (part or the entirety of) the optical element 10b. The VCSEL array may further comprise a submount for accommodating the plurality of emitters. The microlenses 410b, 411b, 412b are shaped and/or positioned to direct the laser radiations in various different directions. In the embodiment shown, the microlens 411b is co-centered with the corresponding active region 411a (so the laser radiation sub-beams 111 generated by the active region 411a are not deflected), while the microlenses 410b and 412b are decentered with respective to the corresponding active regions 410a and 412b in different directions (so the laser radiation sub-beams 110 and 112 generated by the active regions 410a and 412a are deflected into the designed directions).

By switching on and off the individual emitters (active regions) 410, 411, 412, the radiation pattern of the radiation beam 11 can be controlled. In this way, no moving parts are required. Alternatively, a more flexible beam control may be achieved by also moving and/or rotating the microlenses 410b, 411b, 412b in the VCSEL array relative to the active regions 410a, 411a, 412a. A simple adjustment may be achieved by moving the array of microlenses 410b, 411b, 412b (as shown in arrows b in Fig. 5B), by moving the array of active regions 410a, 411a, 412a (as shown in arrows a in Fig 5B), or both. It is also possible to manufacture the microlenses 410b, 411b, 412b as separate parts, and control these elements individually, e.g. using so-called Microelectromechanical systems (MEMS).

For example, as shown in Fig. 5C, for each radiation element 410, 411, 412, the radiation control unit 30 (not shown in Fig. 4C) may be adapted to switch on a radiation element when the effective illumination region for that radiation element is within the predetermined part of the user, and does not switch on the radiation element when the effective illumination region for that radiation element is not within the predetermined part. In the embodiment shown, the predetermined part (the user's face) falls outside the illumination region of the radiation element 412, so this radiation element is turned off. The predetermined part falls within the illumination regions of the radiation elements 410, 411, so these elements are turned on. Note that this schematic figure only shows three radiation elements for simplicity. In practice an LED, EELD or VCSEL array contains many more radiation elements.

Fig. 2 shows the strong wavelength dependence of allowable dose related to eye safety. This means that good control over the emission spectrum of the emitter is important. This can be achieved with the help of secondary optical components, such as filters, e.g. bandpass filters or long-pass filters.

### Multi-user handling and personal dose control

Fig. 6 shows an embodiment in which the electro-optical device 1 further comprises a timer 40 (or this may be included as part of the logic implemented in the radiation control unit 30). This embodiment may comprise one or more measures described above in the context of Figs. 1-5.

In line with this, the timer 40 may start in reaction to an input from the detection unit 20. For example, when the detection unit 20 detects or recognizes a face, it may instruct the timer 40, or to instruct the radiation control unit 30 to start the timer. Once the timer expires, indicating (in conjunction with other information such as the radiant intensity of the radiation beam 11) that a sufficient dose level has been accumulated for a day, the radiation control unit 30 then stops the irradiation of the radiation beam 11 from the radiation unit 10. The timer 40 may be set in accordance with a predetermined expiration time (e.g. two hours), or such that the timer expires after a predetermined dose level (e.g. 10 mJ/cm²) is reached.

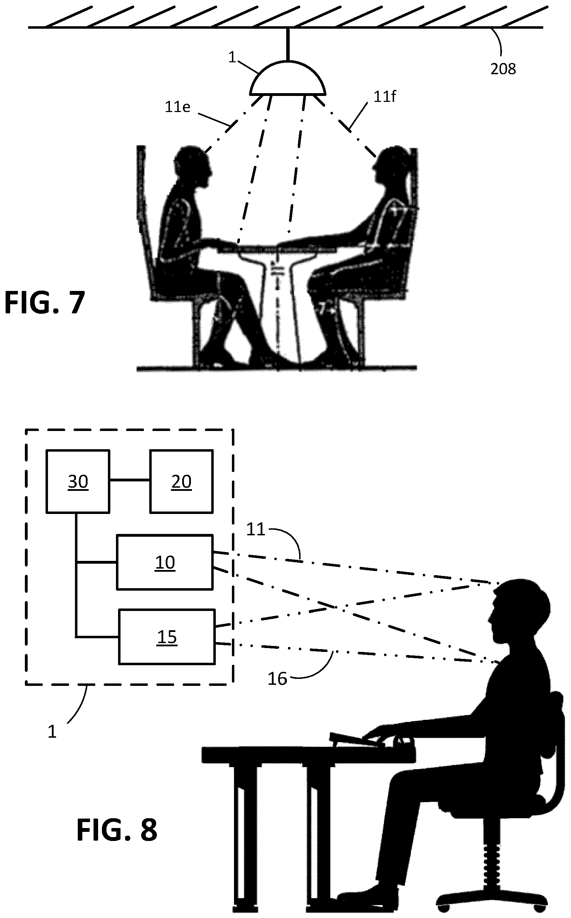

Fig. 7 shows an embodiment of the electro-optical device 1, in which the dose control described in Fig. 6 can be achieved on a user-by-user basis.

In this embodiment, the detection unit is adapted to recognize and distinguish between individual users (e.g. using facial recognition instead of facial detection). The radiation pattern is such that the radiation beam 11 is directed to all the users individually. In the embodiment shown, the radiation beam 11 comprises a plurality of beamlets 11a and 11b, each directed to a predetermined part (e.g. face and/or hands) of a recognized user. The same applies when there are more than two users, in which case there are more than two beamlets. Alternatively or additionally, the radiation beam 11 may rotate between the users under the control of the radiation control unit 30 (not shown in Fig. 7). By taking face detection a step further towards face recognition in this embodiment, the electro-optical device can keep track of each user even if the users happen to move or swap positions.

For each individual user, the electro-optical device 1 may start a timer 40 upon recognizing the user by the detection unit 20, and the radiation control unit 30 may adjust the radiation pattern upon expiration of each individual timer (for a particular user), so that the radiation beam 11 is no longer directed to a user whose accumulated dose has exceeded a certain threshold. For example, in the embodiment shown in Fig. 7, when the user on the left has obtained an accumulated dose exceeding the threshold, the radiation control unit 30 could switch off the radiation beam 11e (e.g. by switching off corresponding LED, EELD or VCSEL emitters, etc).

The radiation control unit 30 may further pause the corresponding timer 40 when the user is no longer recognized (e.g. when the user leaves the room, is temporarily blocked, or moves too far away), and resume the timer when the user is recognized again. As the electro-optical device 1 recognizes each specific user (using racial recognition in this embodiment), this enables the electro-optical device to maintain an accurate dose control, without being interrupted by some users happening to disappear for some time during operation.

In the embodiment shown in Fig. 7, the electro-optical device 1 is incorporated in a general lighting device, but this embodiment is equally applicable to any embodiments of the electro-optical device 1 described above or shown in the Figures.

Further personalization of dose can be achieved by using a wearable device that emits UV-B itself (for example similar to the device shown in Figure 3E) or that only functions as a measurement device that communicates with a UV-B emitting device (e.g. a phone or via a phone). The wearable device can track activity of the user (e.g. detecting whether the user is outside or inside), and/or measure UV-B exposure of the user (by means of a sensor on the wearable device), and/or measure the health status of the user, and/or measure other characteristics of the user such as skin pigmentation, 25(OH)D3 concentration, etc., and based on these inputs determine an optimum personal dose of UV-B radiation for the user.

Fig. 8 shows an embodiment in which the electro-optical device 1 comprises a second radiation unit 15 adapted to generate a second radiation beam 16 in the near-infrared (NIR) region, having a peak emission wavelength between 700-1400 nm for inducing a photobiomodulation (PBM) effect in the user. PBM may be induced by irradiating a user at certain energy/power levels to induce biological or biochemical responses. The irradiation may be in the visible spectrum, such as red light, or in the non-visible spectrum, such as near-infrared (NIR, 700-1400 nm) or infrared (IR). There has been a significant amount of research about the medical benefits of employing PBM therapy to treat physical and psychological symptoms, and also about the health benefits for generally healthy people.

The electro-optical device 1 may include a driver circuit to drive the radiation elements (e.g. LEDs, EELDs or VCSELs) of the second radiation unit 15 with a pulsed driving current, having an amplitude, pulse width and pulse frequency such that the second radiation beam 16 achieves a peak irradiation intensity above 0.1 mW/cm², preferably above 1 mW/cm², but an average irradiation intensity less than 10 mW/cm². Exemplary driver circuits and arrangements to that effect are described in previous applications WO2020/119965 (disclosing the addition of pulsed NIR or IR light to general lighting applications to induce a PBM effect while limiting the energy consumption), WO2021/099642 (disclosing the addition of pulsed red light to general lighting applications to induce a PBM effect), WO2022/200131 (disclosing pulsing arrangements for radiation elements), WO2023/094648 (disclosing arrangements for dose control), WO2024/028203 (disclosing arrangements for radiation beam steering), and WO2025/021895 (disclosing wearable devices).

Certain embodiments described above involve logic operations or calculations. These may be implemented using software (e.g., code embodied on a machine-readable medium or in a transmission signal), hardware, or a combination of these. In software implementation, one or more processors may be used. A hardware implementation may involve the use of dedicated circuitry or logic that is configured to perform certain operations. For example, a hardware module may be a programmable logic device such as a field programmable gate array (FPGA) or an ASIC. It will be appreciated that the decision to implement using software, hardware, or combination of these, may be driven by cost and time considerations.

The descriptions above are intended to be illustrative, not limiting. It will be apparent to the person skilled in the art that alternative and equivalent embodiments of the invention can be conceived and reduced to practice, without departing from the scope of the claims set out below.

## Claims

1. An electro-optical device (1), comprising:
a radiation unit (10) adapted to emit a radiation beam (11) having a peak emission wavelength in a range of 280-320 nm;
a detection unit (20) adapted to detect a distance to a user and generate a detection signal based on the detection; and
a radiation control unit (30) adapted to receive the detection signal and generate a control signal in response;
wherein the electro-optical device (1) is adapted to provide an irradiance in the range 0.003-0.5 mW/cm² to the user.

2. The electro-optical device (1) of claim 1, wherein the electro-optical device (1) is adapted to project the radiation beam (11) in a spread angle within +/- 30°, preferably within +/- 25°, more preferably within +/- 20°, more preferably within +/- 15° about a center line of the radiation beam.

3. The electro-optical device (1) of claim 1 or 2, wherein the electro-optical device (1) is adapted to control the radiation beam based on the control signal to provide a dose in a range of 0.1-200 mJ/cm² to the user.

4. The electro-optical device (1) of any of the preceding claims, wherein the radiation control unit (30) is adapted to adjust a radiant intensity and/or an on-time of the radiation beam (11) emitted by the electro-optical device to provide a predetermined dose to the user.

5. The electro-optical device (1) of any of the preceding claims, wherein the detection unit (20) is adapted to detect a direction of the user, and wherein the detection signal includes an indication of the detected direction.

6. The electro-optical device (1) of claim 5, wherein the electro-optical device (1) is adapted to direct the radiation beam (11) towards the user in dependence on the detected direction.

7. The electro-optical device (1) of any of the preceding claims, wherein the electro-optical device (1) is adapted to adjust a direction, spread-angle, radiation pattern, radiant intensity, and/or on-time of the radiation beam (11) in dependence on the control signal.

8. The electro-optical device (1) of any of the preceding claims, wherein the detection unit (20) is adapted to detect a predetermined part of the user, and wherein the detection signal includes an indication of the detected predetermined part.

9. The electro-optical device (1) of claim 8, wherein the predetermined part covers a substantial portion of the hands of the user, or covers a substantial portion of the face and/or neck of the user, and preferably avoids the eyes of the user.

10. The electro-optical device (1) of any one of claims 8 or 9, wherein the electro-optical device (1) is adapted to reduce a radiant intensity of the radiation beam (11) if the predetermined part of the user includes at least a portion of the user's eyes.

11. The electro-optical device (1) of any one of claims 8-10, wherein the electro-optical device (1) is adapted to adjust a radiation pattern of the radiation beam (11) in dependence on the control signal to reduce a radiant intensity of the radiation beam in a direction of the user's eyes.

12. The electro-optical device (1) of any of the preceding claims, wherein the electro-optical device (1) is adapted to adjust a radiation pattern of the radiation beam (11) by adjusting a direction, spread angle, and/or the radiant intensity of a plurality of portions of the radiation beam.

13. The electro-optical device (1) of any of the preceding claims, wherein the radiation unit (10) comprises an optical element (10b) comprising one or more lenses, and/or one or more mirrors, and/or one or more diffraction optical elements, and wherein the radiation control unit (30) is adapted to move and/or rotate a part of the optical element to adjust a direction of the radiation beam.

14. The electro-optical device (1) of any of the preceding claims, wherein the radiation unit (10) comprises a plurality of radiation elements (410, 411, 412), each driven by a driving current, and wherein the radiation control unit is adapted to adjust the driving current for individual radiation elements to adjust a radiation pattern of the radiation beam (11).

15. The electro-optical device (1) of any of the preceding claims, wherein the radiation unit (10) comprises a plurality of LEDs, and the radiation control unit is adapted to switch on or switch off a subset of the plurality of LEDs to adjust a radiation pattern of the radiation beam (11).

16. The electro-optical device (1) of any of the preceding claims, wherein the detection unit (20) is adapted to distinguish between individual users, and wherein the detection signal includes an indication of the detected individual user.

17. The electro-optical device (1) of any of the preceding claims, wherein the electro-optical device comprises a second radiation unit (15) adapted to emit a second radiation beam (16) having a peak emission wavelength in a range of 610-1400 nm; and
wherein the electro-optical device (1) is adapted to control the second radiation beam based on the control signal to provide a peak irradiation intensity above 0.1 mW/cm² to the user.
